# EUROPÄISCHE PATENTSCHRIFT

(11) **EP 0 064 690 B1**
(45) Veröffentlichungstag und Bekanntmachung des Hinweises auf die Patenterteilung: **13.03.1985**
(21) Anmeldenummer: 82103697.7
(22) Anmeldetag: 30.04.1982
(51) Int. Cl.: C07C 45/49, C07C 47/542, C07C 47/546

(54) **Verfahren zur Herstellung von substituierten Benzaldehyden**
Process for the preparation of substituted benzaldehydes
Procédé de préparation de benzaldéhydes substitués

(30) Priorität: 12.05.1981 DE 3118682
(43) Veröffentlichungstag der Anmeldung: 17.11.1982
(73) Patentinhaber: BAYER AG, 51368 Leverkusen (DE)
(72) Erfinder: Blank, Heinz Ulrich, Dr., D-5068 Odenthal (DE); Wolters, Erich, Dr., D-5162 Niederzier (DE); Neuner, Otto, Dr., D-5060 Bergisch Gladbach 2 (DE)

## Beschreibung

Die Erfindung betrifft ein Verfahren zur Herstellung von substituierten Benzaldehyden durch Umsetzung von substituierten Benzolen mit Kohlenmonoxid und Chlorwasserstoff in Gegenwart von Metallhalogeniden.

Es ist bereits bekannt, beispielsweise Toluol, o-Xylol oder Cumol in Gegenwart von Aluminiumchlorid und Kupfer-I-chlorid bei einer Temperatur von etwa 20 bis etwa 50 °C durch Hindurchleiten von Kohlenmonoxid und Chlorwasserstoff zu den entsprechenden Aldehyden umzusetzen (Ann. 347, 347 (1906)). Weiterhin ist bekannt, daß man auf die Gegenwart des Kupfer-I-chlorids verzichten kann, wenn anstelle des bloßen Hindurchleitens von Kohlenmonoxid bei einem erhöhten CO-Druck, beispielsweise etwa 21 bis 70 bar, gearbeitet wird, wobei Temperaturen von 25 bis 60 °C beschrieben sind (Org. Reactions 5, 290 (1949)). In beiden genannten Literaturstellen ist das Arbeiten in Gegenwart der Sättigungskonzentration von Chlorwasserstoff im organischen Reaktionsmedium beschrieben, die ihrem absoluten Betrage nach sehr klein ist.

Die Literaturstelle org. Reactions weist auf die Schwierigkeiten dieser Reaktion durch Bildung von Nebenprodukten hin. Die zugehörige Tabelle (Seite 300 dieser Literaturstelle) macht diese Schwierigkeiten anhand der wechselnden Ausbeuten für die aufgeführten aromatischen Aldehyde deutlich. Diese Schwierigkeiten werden beim Übergang vom Benzol zu substituierten Benzolen größer, besonders beim Übergang zu alkylsubstituierten Benzolen und hier wiederum insbesondere bei Alkylsubstituenten, die mehr als 1 Kohlenstoffatom enthalten.

Diese Schwierigkeiten durch Nebenproduktbildung und unzureichenden Ausbeuten der als Gattermann-Koch-Reaktion bekannten Umsetzung wird durch weitere Literaturstellen bestätigt : In J. Am. Chem. Soc. 49, 3150 (1927) wird berichtet, daß aus Ethylbenzol unter den Bedingungen der Gattermann-Koch-Reaktion nur Spuren des Ethylbenzaldehydes gefunden wurden. In Ber. 6e 1471 (1933) wird die Herstellung von Cyclohexyl-benzaldehyd in einer Ausbeute von 14 bis 16 % bei Anwendung der drucklosen Variante dieser Reaktion in Gegenwart von Kupfer-I-chlorid beschrieben. US 2158 519 beschreibt die Herstellung von Cuminaldehyd (p-Isopropylbenzaldehyd) bei 25 bis 30 °C und Normaldruck aus Benzol, i-Propylchlorid und Kohlenmonoxid in Gegenwart von AlCl₃/CuCl in einer Ausbeute von nur 25 %. Ebenfalls die Herstellung von Cuminaldehyd wird in J. Am. Chem. Soc. 71,1263 (1949) durch Reaktion von i-Propylbenzol und Kohlenmonoxid bei einem Druck von 35 bar und einer Temperatur von 25 bis 30 °C beschrieben, wobei ein speziell konditioniertes Aluminiumchlorid eingesetzt wird und das Reaktionsgemisch vor Anlegen des CO-Druckes mit HCI-Gas gesättigt wurde. Hierbei wird eine Ausbeute von 40 %, bezogen auf das eingesetzte i-Propylbenzol (Cumol), erreicht, daneben aber eine Reihe von höheralkylierten Nebenprodukten, beispielsweise 31 % Diisopropylbenzaldehyd, gefunden. Diese hohe Menge mehrfach alkylierter Nebenprodukte wurde hierbei erhalten, obwohl zur Zurückdrängung dieser höher alkylierten Produkte pro Mol Isopropylbenzol etwa 2,3 Mol Benzol als Verdünnungsmittel zugesetzt worden waren.

Andere höheralkylierte Benzole, insbesondere solche, die in a-Stellung verzweigt sind, beispielsweise Cyclohexylbenzol oder Cyclopentylbenzol, zeigen ein sehr ähnliches Verhalten. Die oben für den Fall des Cuminaldehyds bereits als negativ angesehene Ausbeute von nur 49 % wird in den Fällen anderer Alkylsubstituenten im allgemeinen noch wesentlich unterschritten.

Diese aufgezeigten Nachteile und geringen Ausbeuten machen die beschriebenen Varianten des Gattermann-Koch-Verfahrens für eine industrielle Anwendung uninteressant, obwohl substituierte aromatische Aldehyde, insbesondere in der Riechstoffindustrie, aber auch als allgemein verwendbare Zwischenprodukte, sehr gefragt sind. So wird beispielsweise in US 2 158 519 Seite 1, rechte Spalte, Zeile 10/11 und Seite 3, rechte Spalte, Zeile 28/29 und in Römpp, Chemie-Lexikon, Franck'sche Verlagshandlung Stuttgart, 6. Auflage 1966, Seite 1284 (Cuminaldehyd) auf die Verwendung in der Riechstoffindustrie hingewiesen. Weiterhin weist DE-OS2817496 auf die Verwendung solcher Aldehyde als wertvolle Ausgangsprodukte für Farbstoffe, Schädlingsbekämpfungsmittel, Kunststoffe und Riechstoffe hin.

Es wurde nun ein Verfahren zur Herstellung von substituierten Benzaldehyden der Formel 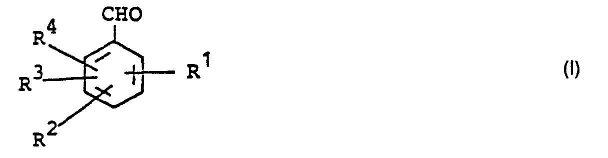 in der
R¹ Alkyl mit mindestens 2 C-Atomen, gegebenenfalls substituiertes Cycloalkyl oder gegebenenfalls substituiertes Benzyl bedeutet und
R² H, gegebenenfalls durch F, CI oder Br substituiertes Phenyl, F, CI oder Br,
R³ H, CH₃, gegebenenfalls durch F, CI oder Br, substituiertes Phenyl oder F, und
R₄ H, F oder CH₃ sein kann, und wobei weiterhin für den Fall, daß R³ und R⁴ benachbart sind, diese gemeinsam Teil eines kondensierten Ringes sein können, durch Umsetzung von substituierten Benzolen der Formel 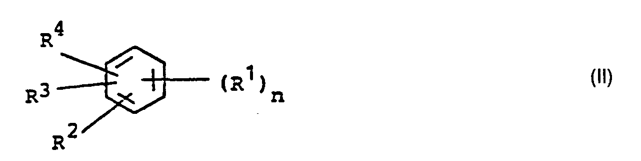 in der
R¹ bis R⁴ die genannte Bedeutung haben, und wobei weiterhin für den Fall, daß R³ und R⁴ benachbart sind, diese gemeinsam Teil eines kondensierten Ringes sein können, und
n die Zahl 1, 2 oder 3 bedeutet,
   mit Kohlenmonoxid und Chlorwasserstoff in Gegenwart von Metallhalogeniden gefunden, das dadurch gekennzeichnet ist, daß man in Gegenwart von 0,5 bis 10 Mol Chlorwasserstoff pro Mol Metallhalogenid bei einem CO-Teildruck von 1 bis 100 bar und einer Temperatur von - 20 °C bis +100°C und gegebenenfalls in einem inerten Lösungsmittel arbeitet und die Umsetzung in Gegenwart eines Benzols der Formel 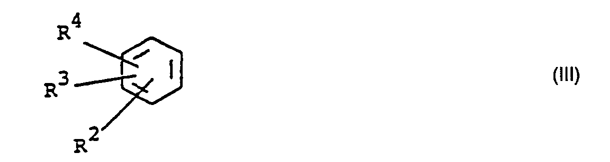 durchführt, in der
R², R³ und R⁴ die angegebene Bedeutung haben.

Als Alkyl mit mindestens 2 C-Atomen sei beispielsweise ein verzweigter oder geradkettiger Rest mit 2 bis 20, bevorzugt 3 bis 10, besonders bevorzugt 3 bis 8 C-Atomen genannt, wie Ethyl, Propyl, Isopropyl, Butyl, Isobutyl, tert.-Butyl, Amyl, Isoamyl, tert.-Amyl, Hexyl, Heptyl, Octyl, Decyl, Dodecyl, Stearyl oder Eikosyl. In bevorzugter Weise sei geradkettiges oder verzweigtes Alkyl ohne tertiäre C-Atome genannt.

Als Cycloalkyl sein ein cycloaliphatischer Rest mit beispielsweise 3 bis 8, bevorzugt 5 bis 6, Ringkohlenstoffatomen genannt, der gegebenenfalls noch mit Methyl oder Ethyl substituiert sein kann, wie Cyclopropyl, Cyclobutyl, Cyclopentyl, Methyl-cyclopentyl, Ethyl-cyclopentyl, Cyclohexyl, Methylcyclohexyl, Ethyl-cyclohexyl, Cycloheptyl und Cyclooctyl.

Als Substituent eines gegebenenfalls substituierten Benzylrestes seien ein oder mehrere Halogene wie F, CI, Br, J, bevorzugt F, CI oder Br, besonders bevorzugt F oder Cl, genannt.

Die Reste R³ und R⁴ können falls sie benachbart sind, Teile eines kondensierten Ringsystems, beispielsweise des Indan-, Tetralin- oder Fluorensystems sein.

Das Zeichen n stellt die Zahl 1, 2 oder 3 dar.

In bevorzugter Weise werden substituierte Benzole der Formel 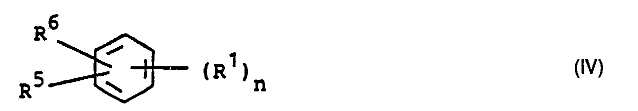 umgesetzt, in der
R¹ und n die oben angegebene Bedeutung haben,
R⁵ für Wasserstoff, Fluor, Chlor, Brom oder gegebenenfalls durch Fluor, Chlor oder Brom substituiertes Phenyl und
R⁶ für Wasserstoff, Fluor oder Methyl steht.

Unter den substituierten Benzolen der Formel (IV) werden weiterhin in besonders bevorzugter Weise solche der Formel 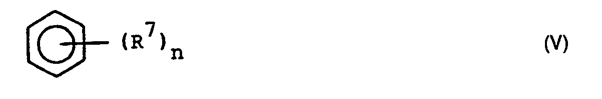 eingesetzt, in der
R⁷ Alkyl mit mindestens 2 C-Atomen gegebenenfalls substituiertes Cycloalkyl oder gegebenenfalls substituiertes Benzyl bedeutet und
n = 1, 2 oder 3 ist.

Ganz besonders bevorzugt umgesetzt werden substituierte Benzole der Formel (V) mit n = 1.

Beispiele für substituierte Benzole für das erfindungsgemäße Verfahren sind : Ethylbenzol, Isopropylbenzol, tert.-Butylbenzol, sec.-Amylbenzol, tert.-Amylbenzol, Methyl-isopropylbenzol, Diisopropylbenzol, Triisopropylbenzol, Cyclopentylbenzol, Cyclohexylbenzol, Diphenylmethan, Fluoren, Indan, Tetralin.

Vorzugsweise : Isopropylbenzol, Diisopropylbenzol, Triisopropylbenzol.

Insbesondere : isopropylbenzol.

Metallhalogenide für das erfindungsgemäße Verfahren sind die auch sonst für die Gattermann-Koch-Reaktion bekannten Stoffe, wie Aluminiumchlorid, Eisen-III-chlorid, Antimonpentachlorid, Zinntetrachlorid, CuCl, CuCl₂, Titantetrachlorid, Zinkchlorid und ähnliche oder Gemische hiervon. Bevorzugt wird Aluminiumchlorid im erfindungsgemäßen Verfahren eingesetzt. Das Metallhalogenid wird in einer Menge von 0,5 bis 2 Mol, bevorzugt 1,0 bis 1,5 Mol, pro Mol substituierter Benzaldehyd der Formel (I), der aufgrund der Einsatzmenge theoretisch erwartet werden kann, eingesetzt.

Erfindungsgemäß wird Chlorwasserstoff in einer Menge von 0,5 bis 10 Mol, bevorzugt 0,7 bis 5 Mol, besonders bevorzugt 1 bis 2 Mol, pro Mol Metallhalogenid eingesetzt. Für die praktische Handhabung des Chlorwasserstoffs, beispielsweise die Überführung aus einer handelsüblichen Stahldruckflasche in einen Druckreaktor zur Durchführung des erfindungsgemäßen Verfahrens, hat es sich als ausreichend herausgestellt, die genannten Mengen an Chlorwasserstoff anhand ihres Teildruckes im Druckgefäß zu messen. Hierbei wurde gefunden, daß beim Befüllen eines Druckreaktors im Temperaturbereich von etwa 0 °C bis Zimmertemperatur zur anschließenden Durchführung des erfindungsgemäßen Verfahrens den angegebenen molaren Mengen an Chlorwasserstoff etwa 0,5 bis 30 bar, bevorzugt etwa 1 bis 15 bar, besonders bevorzugt etwa 2 bis 5 bar HCI-Teildruck entsprechen.

Das erfindungsgemäße Verfahren kann beispielsweise bei einem Kohlenmonoxid-Teildruck von 1 bis 100 bar durchgeführt werden. Bevorzugt wird ein CO-Teildruck von 2 bis 20 bar, besonders bevorzugt 3 bis 10 bar, eingestellt.

Die Reaktionstemperatur kann in einem weiten Bereich schwanken, beispielsweise von - 20 °C bis + 100 °C. Bevorzugt wird bei - 15 bis + 50 °C, besonders bevorzugt bei - 10 bis + 20 °C und ganz besonders bevorzugt bei - 5 bis + 15 °C gearbeitet.

Das erfindungsgemäße Verfahren kann in einem inerten Lösungsmittel durchgeführt werden. Als Beispiele für solche inerten Lösungsmittel seien genannt : halogenierte Kohlenwasserstoffe, wie Dichlorethan, Trichlorethan, Tetrachlorethan oder Methylenchlorid, weiterhin Schwefelkohlenstoff oder Nitrobenzol. Da eine über den o. g. Bereich hinausgehende Menge von Chlorwasserstoff für das erfindungsgemäße Verfahren unschädlich ist, kann das erfindungsgemäße Verfahren jedoch auch statt in einem der genannten inerten Lösungsmittel in überschüssigem flüssigem Chlorwasserstoff durchgeführt werden.

Das erfindungsgemäße Verfahren wird in Gegenwart eines substituierten Benzols der Formel (III) durchgeführt. Dieses substituierte Benzol der Formel (III) unterscheidet sich von dem zu formylierenden substituierten Benzol der Formel (11) durch das Fehlen des gegebenenfalls n-fach vorhandenen Substituenten R¹. Für den Fall, daß in bevorzugter Weise anstelle eines substituierten Benzols der Formel (11) solche der o. g. Formel (IV) oder der Formel (V) erfindungsgemäß eingesetzt werden, wird die Umsetzung in entsprechender Weise in Gegenwart eines substituierten Benzols durchgeführt, bei dem ebenfalls der Substituent R¹ entsprechende Substituent R⁷ fehlt.

Dieses substituierte Benzol der Formel (III) bzw. die diesem entsprechenden und soeben beschriebenen substituierten Benzole, die sich von der Formel (IV) ebenfalls durch das Fehlen des Substituenten R¹ und von der Formel (V) durch das Fehlen des Substituenten R⁷ ableiten, werden im erfindungsgemäßen Verfahren beispielsweise in einer Menge von 2 bis 20 Mol, bevorzugt 2 bis 10 Mol, besonders bevorzugt 3 bis 5 Mol pro Mol Verbindung der Formel (11), Verbindung (IV) bzw. Verbindung (V) zusätzlich zu der Menge eingesetzt, die von der Stöchiometrie her erforderlich ist, um aus der Verbindung der Formel (11), der Formel (IV) bzw. der Formel (V) mit n-facher Substitution die entsprechende Verbindung mit nur 1- facher Substitution zu gewinnen. Dies soll am Beispiel der Verbindungen der Formeln (11) und (III) durch die folgende Formelgleichung näher erläutert werden :

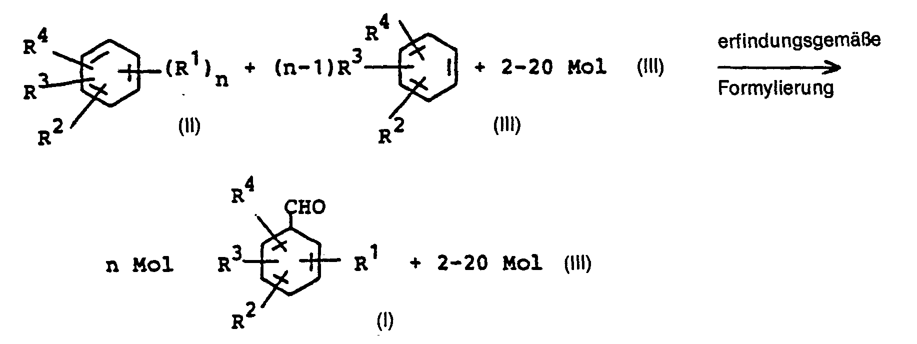

Beispielsweise werden für den Fall, daß der Index n in der Formel (11) die Bedeutung 1 hat, 2 bis 20 Mol der Verbindung der Formel (III) eingesetzt; für den Fall, daß n = 2 ist, werden 3 bis 25 Mol der Verbindung der Formel (III) eingesetzt und für den Fall, daß n = 3 ist, werden 4 bis 22 Mol der Verbindung der Formel (III) eingesetzt. Diese zugesetzte Menge an substituiertem Benzol der Formel (III) bzw. die diesem entsprechende von den Formeln (IV) bzw. (V) abgeleiteten substituierten Benzole dienen als Reaktionsmedium für die erfindungsgemäße Formylierung. Daneben kann jedoch, beispielsweise für den Fall daß die umzusetzenden Verbindungen und die als Reaktionsmedium vorgesehenen Verbindungen fest sind, eines oder mehrere der o. g. Verdünnungsmittel als Reaktionsmedium zugefügt werden. Die Menge dieses Verdünnungsmittels ist so zu bemessen, daß ein homogenes flüssiges Reaktionsgemisch bei der gewählten Reaktionstemperatur vorliegt. Beispielsweise wird das zugesetzte Verdünnungsmittel bzw. ein Gemisch von Verdünnungsmitteln in einer Menge von 0,5 bis 15 Mol pro Mol des Gemisches aus Verbindungen der Formeln (11) und (III) bzw. der von diesen Formeln abgeleiteten bevorzugten Reaktionspartner angewendet.

Es wurde weiterhin gefunden, daß es ohne Beeinträchtigung der erfindungsgemäßen Formylierung möglich ist, das zu formylierende substituierte Benzol der Formel (11), (IV) oder (V), im Reaktionsgemisch durch Alkylierung eines substituierten Benzols der Formel (III) bzw. der aus (IV) oder (V) durch Fortlassen der Reste R¹ bzw. R⁷ entsprechend abgeleiteten Benzols mit einem Alkylierungsmittel herzustellen, das zur Einführung des Restes R¹ bzw. R⁷ geeignet ist. Als solche Alkylierungsmittel seien beispielsweise das dem Rest R¹ bzw. R⁷ entsprechende Chlorid oder bei entsprechendem molekularen Aufbau des Restes R¹ bzw. R⁷ ein diesem Rest R¹ bzw. R⁷ zugrundeliegendes Olefin genannt. Als Alkylierungsmittel sei weiterhin ein Benzol der Formel (II), (IV) oder (V) genannt, bei dem n größer als 1 ist und das einen oder mehrere Reste R¹ bzw. R⁷ übertragen kann. Die Menge des dem Rest R¹ bzw. R⁷ zugrundeliegenden Chlorids oder Olefins wird so bemessen, wie es weiter oben für das molare Verhältnis von Metallhalogenid und dem formylierenden substituierten Benzol beschrieben ist, beispielsweise 0,5 bis 2,0 Mol des Metallhalogenids pro Mol des dem Substituenten R¹ bzw. R⁷ zugrundeliegenden Chlorids oder Olefins.

Die zuletzt genannte Reaktionsvariante wird bevorzugt unter Benutzung eines dem Rest R¹ bzw. R⁷ zugrundeliegenden Olefins oder eines Benzols der Formel (II), (IV) oder (V) durchgeführt.

Die Reihenfolge des Zusammengebens der Reaktionsteilnehmer ist weitgehend beliebig. Aus Gründen der Handhabung kann es vorteilhaft sein, das feste Metallhalogenid vorzulegen, während die flüssigen, gelösten oder gasförmigen Stoffe in beliebiger Reihenfolge, beispielsweise auch in den bereits geschlossenen Druckreaktor eingepumpt werden können.

So kann das erfindungsgemäße Verfahren beispielsweise in der Weise durchgeführt werden, daß in einem rostfreien Stahlautoklaven, der mit einer Rührvorrichtung ausgerüstet ist, der Katalysator, die Ausgangssubstanz und gegebenfalls das Verdünnungsmittel vorgelegt werden. Unter Rühren wird sodann die gewählte Temperatur eingestellt und über eine fest angeschlossene Leitung flüssiger Chlorwasserstoff bis zum gewünschten Druck eingepumpt. Anschließend wird, beispielsweise aus einer Stahldruckflasche über ein Reduzierventil, bis zum entsprechenden Druck Kohlenmonoxid zugefügt und dieser Druck bis zum Ende der Reaktion aufrechterhalten. Durch eine Kühlvorrichtung wird gegebenfalls die Reaktionswärme in dem für die Einhaltung der Temperatur erforderlichen Maße abgeführt. Die Reaktion ist im allgemeinen beendet, wenn kein Kohlenmonoxid mehr verbraucht wird, was am konstant bleibenden Druck des Reaktionsgemisches erkennbar ist. Bei sonst gleicher Arbeitsweise kann jedoch auch das Ausgangsprodukt, gegebenenfalls mit Verdünnungsmittel, in das Gemisch der anderen bereits vorgelegten Komponenten eingepumpt werden.

Für den Fall, daß in der beschriebenen Weise von einem R¹⁻ bzw. R⁷⁻freien Benzol und dem Olefin ausgegangen werden soll, das dem Rest R¹ bzw. R⁷ zugrundeliegt, kann ein solches Olefin beispielsweise vor der Zugabe des Kohlenmonoxids, gegebenenfalls auch vor der Zugabe des Chlorwasserstoffs, in das Reaktionsgemisch eingeführt werden.

Für den Fall, daß von dem R^{l-} bzw. R⁷⁻freien Benzol und dem Chlorid ausgegangen werden soll, das dem Rest R¹ bzw. R⁷ zugrundeliegt, kann dieses Chlorid beispielsweise nach Vorlegen des Katalysators und des R¹⁻ bzw. R⁷⁻frelen Benzols in das Reaktionsgemisch gegeben werden. Bei dieser Reaktionsvariante wird vorteilhafterweise der Chlorwasserstoff erst nach der Zugabe des dem Rest R¹ bzw. R⁷ zugrundeliegenden Chlorids in das Reaktionsgemisch gegeben. Da in diesem Fall bei der Alkylierung des R¹⁻ bzw. R⁷⁻freien substituierten Benzols pro Mol des dem Rest R¹ bzw. R⁷ zugrundeliegenden Chlorids 1 Mol Chlorwasserstoff frei wird, kann auf die Zugabe von Chlorwasserstoff völlig verzichtet werden, da dieses infolge der beschriebenen Alkylierung bereits in einer Menge im Reaktionsgemisch vorliegt, die erfindungsgemäß ausreichend ist. Jedoch ist es erfindungsgemäß durchaus möglich, über die aus der Alkylierung entstehende Menge Chlorwasserstoff hinaus weiteren Chlorwasserstoff im Rahmen der beschriebenen Mengen zum Reaktionsgemisch zuzusetzen. Die weitere Durchführung geschieht sodann in der oben beschriebenen Weise.

In einer bevorzugten Durchführungsform werden in einem Autoklaven mit Rührvorrichtung das Metallhalogenid, der Chlorwasserstoff und ein zur Bildung eines teilweise flüssigen Reaktionsmediums geeigneter Reaktionsteilnehmer vorgelegt, der gewünschte CO-Druck eingestellt und die Reaktion durch Zupumpen des zu formylierende substituierten Benzols oder eines der oben genannten Alkylierungsmittel, die das zu formylierende substituierte Benzol bilden, in Gang gesetzt und weitergeführt. Der Gesamtdruck wird hierbei durch kontinuierliches Nachdosieren von CO aufrechterhalten. Wird ein substituiertes Benzol der Formel (11), bei dem R¹ = H ist, formyliert, wird zur Bildung eines teilweise flüssigen Reaktionsmediums eines der genannten inerten Lösungsmittel und/oder flüssiger Chlorwasserstoff eingesetzt. Wird ein substituiertes Benzol der Formel (11) formyliert, wird zur Bildung eines teilweise flüssigen Reaktionsmedium das entsprechende Benzol (111) sowie gegebenenfalls überschüssiger flüssiger Chlorwasserstoff und gegebenenfalls zusätzlich ein inertes Lösungsmittel eingesetzt. Sodann wird zur Bildung des zu formylierenden substituierenden Benzols eines der genannten Alkylierungsmittel zugepumpt, also ein dem Rest R¹ zugrundeliegendes Chlorid oder Olefin oder Benzole der Formel (11), in denen n größen als 1 ist; auch durch Zupumpen von Benzol der Formel (11) mit n = 1 allein oder im Gemisch mit den genannten Alkylierungsmitteln kann die Reaktion in diesem Fall in Gang gesetzt werden.

Beispielsweise wird zur Herstellung von Cuminaldehyd nach dieser bevorzugten Reaktionsform AICI₃, HCI und Benzol in den beschriebenen Verhältnissen und Mengen vorgelegt, der gewünschte CO-Druck aufgepreßt und sodann unter Rühren, Propen, Isopropylchlorid, Cumol, Diisopropylbenzol, Triisopropylbenzol oder ein Gemisch aus diesen zugepumpt.

Zur Aufarbeitung wird der Autoklav entspannt und sein Inhalt zur Zersetzung des Katalysators in Eiswasser gegossen. Die organische Phase wird abgetrennt, gegebenenfalls durch Waschen von anorganischen Stoffen befreit und nach dem Entfernen des Verdünnungsmittels in üblicher Weise, beispielsweise durch weitere Destillation oder durch Kristallisation auf den substituierten Benzaldehyd der Formel (I) als Reaktionsprodukt aufgearbeitet.

Das erfindungsgemäße Verfahren löst die Aufgabe, Reaktionsbedingungen zu finden, die die Bildung der höher alkylierten produkte zugunsten der Monoalkylbenzaldehyde unterdrücken und so eine wirtschaftliche Nutzung der Gattermann-Koch-Reaktion für diese Aldehyde ermöglichen. Im allgemeinen tritt die erfindungsgemäß einzuführende Aldehydgruppe in p-Stellung zum Alkylrest ein. Das erfindungsgemäße Verfahren ist also durch eine sehr hohe Selektivität in bezug auf die Anzalhl der Alkylgruppen und die Stellung der einzuführenden Aldehydgruppe gekennzeichnet.

Nach den Angaben des Standes der Technik war der Chlorwasserstoff immer in einer Menge im Reaktionsgemisch anwesend. die seiner geringer Sättigungskonzentration in diesem Reaktionsgemisch entspricht. Es ist überraschend, daß durch die erfindungsgemäße Erhöhung der Chlowasserstoffmenge im Reaktionsgemisch die gefundenen Vorteile erreicht werden konnten.

Ein weiterer überraschender Effekt der erfindungsgemäßen Chlorwasserstoffmenge im Reaktionsgemisch ist die unerwartet hohe Steigerung der Reaktivität. dies bedeutet, daß sich die Reaktionszeit verkürzt, daß die Reaktion auch bei sehr niedrigen Kohlenmonoxiddrucken, beispielsweise schon bei 2 bis 3 bar, auch ohne die Verwendung von Hilfsmitteln wie Kupfer-I-chlorid zügig abläuft und daß auch bei Temperaturen unter 0 °C, beispielsweise bei - 10°C, noch eine Reaktion stattfindet. Ein weiterer überraschender Vorteil der erfindungsgemäßen hohen Menge von Chlorwasserstoff besteht in der Möglichkeit, substituierte Benzole mit einer höheren Anzahl an Alkylgruppen, wie sie bei technischen Aklylierungen erhalten werden, ohne Ausbeuteverluste in die erfindungsgemäße Reaktion einzusetzen. Beispielsweise betrifft dies den Einsatz von 2-fach und/oder 3-fach isopropylierten und gegebenenfalls zusätzlich substituierten Benzolen. Dies war bisher bei 3-fach alkylierten benzolen nicht der Fall und auch bei 2-fach alkylierten Benzolen nur bei Hinnahme von Ausbeuteverlusten an monoalkylierten Benzaldehyd.

### Beispiele 1 bis 10

170,0 g (1,41 Mol) Cumol, 327 g (4,19 Mol) Benzol, 255 g (1,91 Mol) Aluminiumchlorid werden in einem 1,3 Liter-V4A-Stahlautoklaven eingefüllt und unter Rühren auf die in der Tabelle angegebene Temperatur T (°C) gebracht. Anschließend wird die in der Tabelle angegebene Menge an flüssiqem Chlorwasserstoff eingepumpt. Dem entstandenen HCI-Druck P_{HCI} (bar) wird der angegebene Kohlenmonoxid-Druck P_{co} (bar) überlagert und der resultierende Gesamtdruck bis zur Beendigung der Kohlenmonoxidaufnahme in der angegebenen Reaktionszeit t (h) durch Nachdrücken von Kohlenmonoxid aufrechterhalten. Nach dem Belüften wird der Autoklaveninhalt auf Eiswasser gegossen, die organische Phase isoliert, destilliert und die Ausbeute an Monoisopropylbenzaldehyd und der Summe der Di- und Triisopropylbenzaldehyde bestimmt und in Prozent der theoretischen Ausbeuten, bezogen auf eingesetztes Cumol, angegeben. Der Monoisopropylbenzaldehyd enthält 97 bis 98 % 4-Isopropylbenzaldehyd und 2 bis 3 % 2- und 3-Isopropylbenzaldehyd. Daneben entstehen im wesentlichen 2,4-Diisopropylbenzaldehyd und 2,4,6-triisopropylbenzaldehyd.

Die Beispiele 1-3 sind nicht erfindungsgemäß (Vergleichsbeispiele).

### (Siehe Tabelle Seite 7 f.)

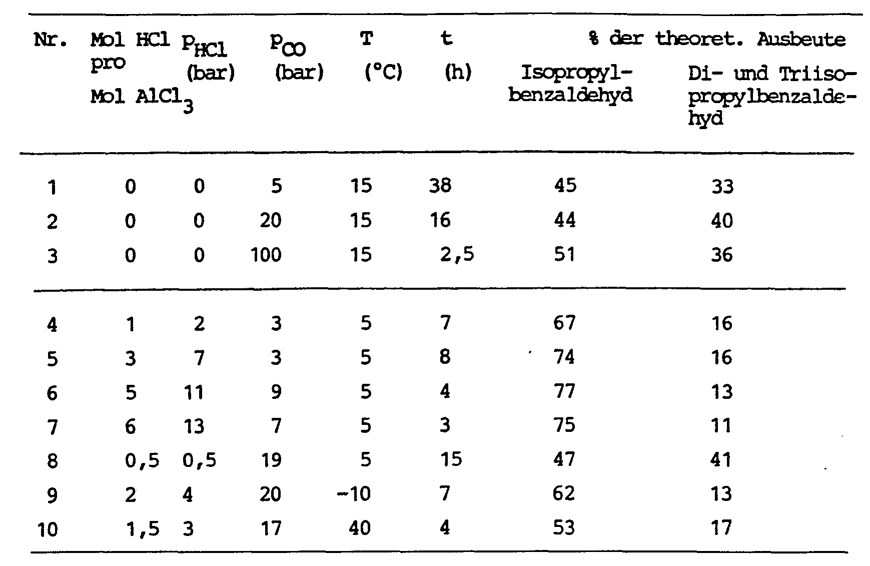

### Beispiel 11

Zu 204,0 g (1,53 Mol) AICI₃ werden in einen 1,3 Liter-V4A-Stahlautoklaven 300 ml (7,0 Mol) flüssiger Chlorwasserstoff eingepumpt. Dem Chlorwasserstoffdruck von 27 bar bei 3°C wird dann ein CO-Druck von 12 bar überlagert, so daß der Gesamtdruck 39 bar beträgt. Im Verlauf von 50 Minuten wird unter Rühren Cumol eingepumpt. Die Temperatur wird durch entsprechende Kühlung bei ca. 5°C gehalten. Nach 10-minütiger Nachreaktionszeit wird wie bei Beispiel 1 bis 10 aufgearbeitet. Die Ausbeute an Monoisopropylbenzaldehyd beträgt 58 % der theoretischen Ausbeute. Daneben werden 28 % an Di- und Triisopropylbenzaldehyden erhalten.

### Beispiele 12 bis 15

Die in der Tabelle angegebenen Mengen an Di-(DB)- bzw. Triisopropylbenzol (TB) und Benzol (B) werden wie im Beispiel 1 bis 10 behandelt. Die Ergebnisse können der Tabelle entnommen werden. Beispiel 12 dient als Vergleichsbeispiel.

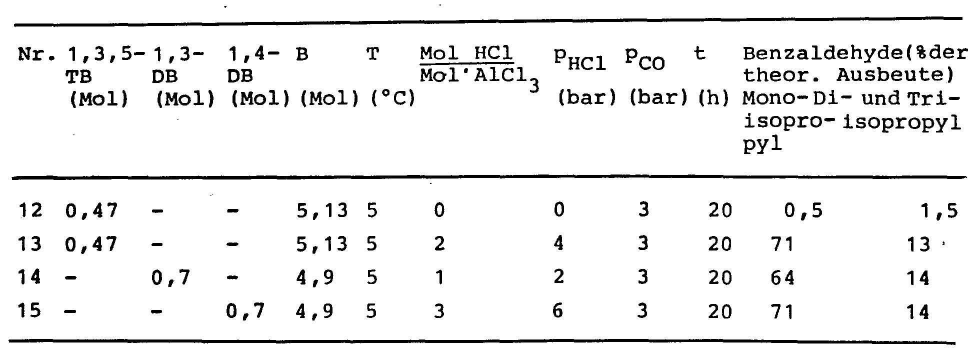

### Beispiele 16 bis 19

Je 1,4 Mol der in der Tabelle angeführten Ausgangssubstanzen wird wie in den Beispielen 1-10 bei 5 °C und jeweils 5 bar Chlorwasserstoffdruck und 5 bar Kohlenmonoxiddruck umgesetzt. Die Ausbeuten an Monoalkylbenzaldehyd bzw. die Summe der Di- und Trialkylbenzaldehyde sind aus der Tabelle ersichtlich.

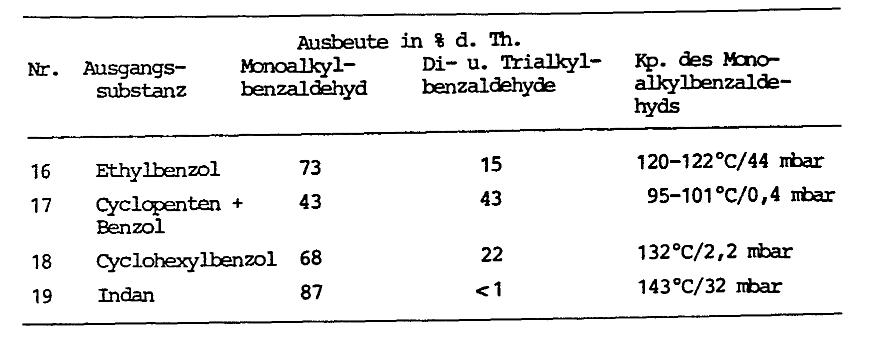

### Beispiel 20

452 g (5,8 Mol) Benzol, 255 g (1,91 Mol) AlC1₃ werden im Autoklaven vorgelegt. Bei 5 °C wird unter Rühren Chlorwasserstoff bis zu einem Druck von 7 bar eingepumpt. Diesem Druck wird ein druck von 3 bar Kohlenmonoxid überlagert. Dann wird im Verlauf einer Stunde 58,9 g (1,4 Mol) Propen eingepumpt. Danach wird der Gesamtdruck von 10 bar durch Nachdrücken von CO so lange aufrechterhalten, bis keine CO-Aufnahme mehr erfolgt. Dies nimmt etwa 24 Stunden in Anspruch. Die Aufarbeitung geschieht wie in den Beispielen 1 bis 10. Man erhält 73% Monoisopropylbenzaldehyd und 18% Di- und Triisopropylbenzaldehyd, jeweils bezogen auf die theoretische Ausbeute.

### Beispiel 21

452 g (5,8 Mol) Benzol und 255 g (1,91 Mol) AICI₃ werden im Autoklaven vorgelegt. Bei 5 °C werden bei einem CO-Druck von 6 bar ein Verlauf einer Stunde unter Rühren 110 g (1,4 Mol) Isopropylchlorid eingepumpt. Über ein Reduzierventil wird CO in dem Maße nachgeführt, daß der Druck konstant 6 bar beträgt. Nach 18 h wird kein CO mehr aufgenommen. Die Aufarbeitung geschieht wie im vorigen Beispiel. Man erhält 157 g Monoisopropylbenzaldehyd. Dies entspricht 75 % der Theorie. Daneben werden 16 % der Theorie an Di- und Tri-isopropylbenzaldehyd erhalten.

## Patentansprüche

1. Verfahren zur Herstellung von substituierten Benzaldehyden der Formel 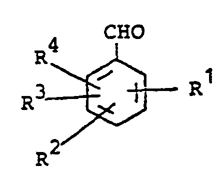 in der
R¹ Alkyl mit mindestens 2 C-Atomen, gegebenenfalls substituiertes Cycloalkyl oder gegebenenfalls substituiertes Benzyl bedeutet und
R² = H, gegebenenfalls durch F, CI oder Br substituiertes Phenyl, F, Cl oder Br,
R³ = H, CH₃, gegebenenfalls durch F, CI oder Br substituiertes Phenyl oder F, und
R⁴ = H, F oder CH₃ bedeutet,
wobei weiterhin für den Fall, daß R³ und R⁴ benachbart sind, diese gemeinsam Teil eines kondensierten Ringes sein können, durch Umsetzung von substituierten Benzolen der Formel 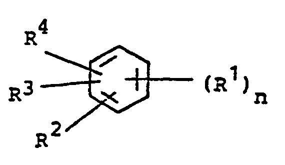 in der
R¹ bis R⁴ die genannte Bedeutung haben und
n die Zahl 1, 2 oder 3 bedeutet,
mit Kohlenmonoxid und Chlorwasserstoff in Gegenwart von Metallhalogeniden, dadurch gekennzeichnet, daß man in Gegenwart von 0,5 bis 10 Mol Chlorwasserstoff pro Mol Metallhalogenid bei einem Kohlenmonoxid-Teildruck von 1 bis 100 bar und einer Temperatur von - 20 °C bis + 100 °C und gegebenenfalls in einem inerten Verdünnungsmittel arbeitet und die Umsetzung in Gegenwart eines Benzols der Formel 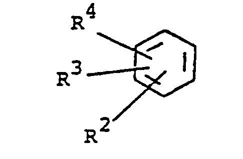 durchführt, in der
R², R³ und R⁴ die angegebene Bedeutung haben.

2. Verfahren nach Anspruch 1, dadurch gekennzeichnet, daß man 0,7 bis 5 Mol Chlorwasserstoff pro Mol Metallhalogenid einsetzt.

3. Verfahren nach Ansprüchen 1 und 2, dadurch gekennzeichnet, daß man 1 bis 2 Mol Chlorwasserstoff pro Mol Metallhalogenid einsetzt.

4. Verfahren nach Ansprüchen 1 bis 3, dadurch gekennzeichnet, daß ein CO-Teildruck von 2 bis 20 bar eingestellt wird.

5. Verfahren nach Ansprüchen 1 bis 4, dadurch gekennzeichnet, daß eine Temperatur von - 15 bis + 50 °C eingestellt wird.

6. Verfahren nach Ansprüchen 1 bis 5, dadurch gekennzeichnet, daß eine Temperatur von - 10 bis + 20 °C eingestellt wird.

7. Verfahren nach Ansprüchen 1 bis 6, dadurch gekennzeichnet, daß eine Temperatur von - 5 bis + 15 °C eingestellt wird.

8. Verfahren nach Ansprüchen 1 bis 7, dadurch gekennzeichnet, daß man (n - 1) Mol und zusätzlich weitere 2 bis 20 Mol des Benzols der Formel 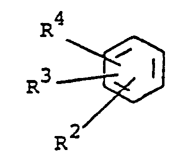 pro Mol des Benzols der Formel 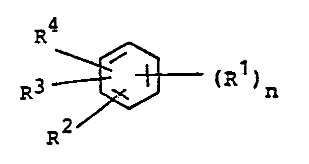 einsetzt, wobei
n und die Reste R¹ bis R⁴ die in Anspruch 1 gegebene Bedeutung haben.

9. Verfahren nach Ansprüchen 1 bis 8, dadurch gekennzeichnet, daß zur Alkylierung eines Benzols der Formel 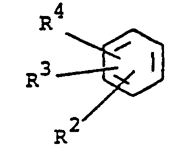 in der
R² für H, gegebenenfalls durch F, CI oder Br substituiertes Phenyl, F, CI oder Br,
R³ für H, CH₃, gegebenenfalls durch F, CI oder Br substituiertes Phenyl oder F steht und
R⁴ H, F oder Ch₃ bedeutet,
simultan zur Formylierung als Alkylierungsmittel ein dem Rest R¹ entsprechendes Chlorid oder Olefin oder ein Benzol der Formel 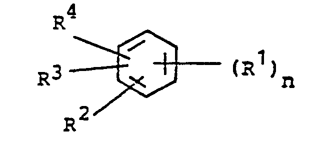 in der
R₁ Alkyl mit mindestens 2 C-Atomen, Cycloalkyl oder gegebenenfalls substituiertes Benzyl bedeutet,
R², R³ und R⁴ den angegebenen Ümfang haben und
n 2 oder 3 bedeutet,
eingesetzt wird.

10. Verfahren nach Anspruch 9, dadurch gekennzeichnet, daß als Alkylierungsmittel ein dem Rest R¹ entsprechendes Olefin oder ein Benzol der Formel 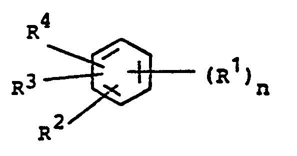 in der
R¹ Alkyl mit mindestens 2 C-Atomen, Cycloalkyl oder gegebenenfalls substituiertes Benzyl bedeutet,
R², R³ und R⁴ den angegebenen Umfang haben und
n 2 oder 3 bedeutet,
eingesetzt wird.

## Claims

1. Process for the preparation of substituted benzaldehydes of the formula 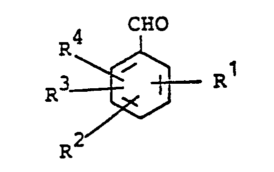 in which
R¹ denotes alkyl with at least 2 C atoms, optionally substituted cycloalkyl or optionally substituted benzyl and
R² is H, F, Cl, Br, or phenyl optionally substituted by F, CI or Br,
R³ is H, CH₃, F or phenyl optionally substituted by F, CI or Br and
R⁴ is H, F or CH₃,
and furthermore, in the case where R³ and R⁴ are adjacent, these can together be part of a fused ring, by reacting substituted benzenes of the formula 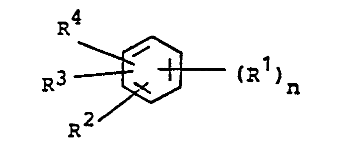 in which
R¹ to R⁴ have the meaning given, and
n denotes the number 1, 2 or 3
with carbon monoxide and hydrogen chloride in the presence of metal halides, characterised in that the reaction is performed in the presence of 0.5 to 10 mols of hydrogen chloride per mol of metal halide under a partial pressure of carbon monoxide from 1 to 100 bars and at a temperature from - 20 °C to + 100 °C, and, if appropriate, in an inert diluent, and the reaction is carried out in the presence of a benzene of the formula 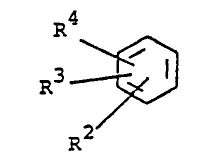 in which
R², R³ and R⁴ have the meaning given.

2. Process according to Claim 1, characterised in that 0.7 to 5 mols of hydrogen chloride per mol of metal halide are employed.

3. Process according to Claims 1 and 2, characterised in that 1 to 2 mols of hydrogen chloride per mol of metal halide are employed.

4. Process according to Claims 1 to 3, characterised in that a partial pressure of CO from 2 to 20 bars is set.

5. Process according to Claims 1 to 4, characterised in that a temperature of from - 15 to + 50 °C is set.

6. Process according to Claims 1 to 5, characterised in that a temperature from - 10 to + 20 °C is set.

7. Process according to Claims 1 to 6, characterised in that a temperature from - 5 to + 15 °C is set.

8. Process according to Claims 1 to 7, characterised in that (n - 1) mols and an additional 2 to 20 mols of the benzene of the formula 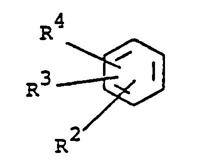 per mol of the benzene of the formula 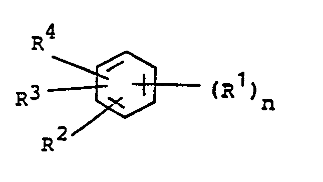 wherein
n and the radicals R¹ to R⁴ have the meaning given in Claim 1,
are employed.

9. Process according to Claims 1 to 8, characterised in that, for the alkylation of a benzene of the formula 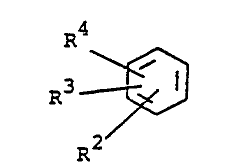 in which
R² represents H, F, CI, Br, or phenyl optionally substituted by F, CI or Br,
R³ represents H, CH₃, F or phenyl optionally substituted by F, CI or Br and
R⁴ is H, F or CH₃,
at the same time as formylation, a chloride or olefine or a benzene corresponding to the radical R¹ is employed as an alkylating agent, this benzene having the formula 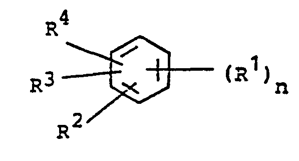 in which
R¹ denotes alkyl with at least 2 C atoms, cycloalkyl or optionally substituted benzyl,
R², R³ and R⁴ have the range given and
n denotes 2 or 3.

10. Process according to Claim 9, characterised in that the alkylating agent employed is an olefine or a benzene corresponding to the radical R¹, the benzene having the formula 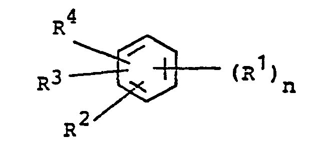 in which
R¹ denotes alkyl with at least 2 C atoms, cycloalkyl or optionally substituted benzyl,
R², R³ and R⁴ have the range given and
n denotes 2 or 3.

## Revendications

1. Procédé de production de benzaldéhydes substitués de formule 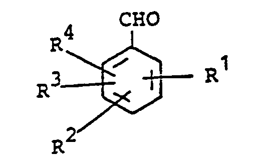 dans laquelle
R¹ est un groupe alkyle ayant au moins 2 atomes de carbone, un groupe cycloalkyle éventuellement substitué ou un groupe benzyle éventuellement substitué et
R² = H, phényle éventuellement substitué par F, CI ou Br, le fluor, le chlore ou le brome,
R³ = H, CH₃, phényle éventuellement substitué par F, CI ou Br, ou le fluor, et
_{R}⁴ = H, F ou CH₃,
en outre, au cas où R³ et R⁴ sont voisins, ils forment ensemble une partie d'un noyau condensé, par réaction de benzènes substitués de formule 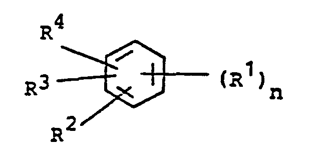 dans laquelle
R¹ à R⁴ ont la définition indiquée ci-dessus et
n représente le nombre 1, 2 ou 3,
avec l'oxyde de carbone et le chlorure d'hydrogène en présence d'halogénures métalliques, caractérisé en ce qu'on opère en présence de 0,5 à 10 moles de chlorure d'hydrogène par mole d'halogénure métallique à une pression partielle d'oxyde de carbone de 1 à 100 bars et à une température de - 20 °C à + 100 °C et, le cas échéant, dans un diluant inerte, et on conduit la réaction en présence d'un benzène de formule 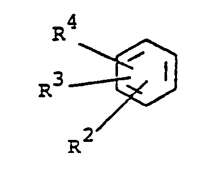 dans laquelle
R², R³ et R⁴ ont la définition indiquée.

2. Procédé suivant la revendication 1, caractérisé en ce qu'on utilise 0,7 à 5 moles de chlorure d'hydrogène par mole d'halogénure métallique.

3. Procédé suivant les revendications 1 et 2, caractérisé en ce qu'on utilise 1 à 2 moles de chlorure d'hydrogène par mole d'halogénure métallique.

4. Procédé suivant les revendications 1 à 3, caractérisé en ce qu'on règle une pression partielle de CO de 2 à 20 bars.

5. Procédé suivant les revendications 1 à 4, caractérisé en ce qu'on règle une température de - 15 à + 50 °C.

6. Procédé suivant les revendications 1 à 5, caractérisé en ce qu'on règle une température de - 10 à + 20 °C.

7. Procédé suivant les revendications 1 à 6, caractérisé en ce qu'on règle une température de - 5 à + 15 °C.

8. Procédé suivant les revendications 1 à 7, caractérisé en ce qu'on utilise (n - 1) moles et en outre, encore 2 à 20 moles du benzène de formule 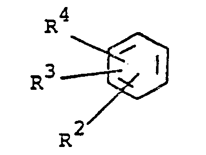 par mole du benzène de formule 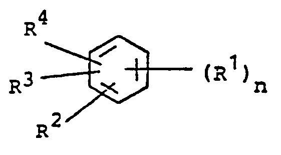 n et les restes R¹ à R⁴ ayant la définition indiquée dans la revendication 1.

9. Procédé suivant les revendications 1 à 8, caractérisé en ce qu'on utilise comme agent alkylant pour l'alkylation d'un benzène de formule 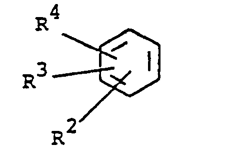 dans laquelle
R² représente H, un groupe phényle éventuellement substitué par F, Clou Br, le fluor, le chlore ou le brome,
R³ représente H, CH₃, un groupe phényle éventuellement substitué par F, Clou Br ou le fluor et
R⁴ représente H, F ou CH₃,
en même temps que pour la formylation, un chlorure ou une oléfine correspondant au reste R¹ ou un benzène de formule 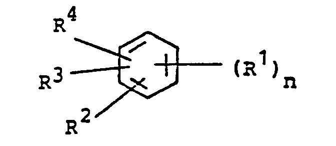 dans laquelle
R¹ est un groupe alkyle ayant au moins 2 atomes de carbone, cycloalkyle ou benzyle éventuellement substitué,
R², R³ et R⁴ ont la définition indiquée et
n a la valeur 2 ou 3.

10. Procédé suivant la revendication 9, caractérisé en ce qu'on utilise comme agent alkylant une oléfine correspondant au reste R¹ ou un benzène de formule 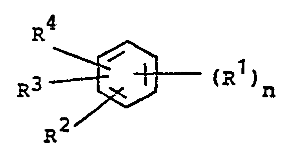 dans laquelle
R¹ est un groupe alkyle ayant au moins 2 atomes de carbone, cycloalkyle ou benzyle éventuellement substitué,
R², R³ et R⁴ ont la définition indiquée et
n a la valeur 2 ou 3.
